Europäisches Patentamt

European Patent Office

Office européen des brevets

(11) Publication number: **0 557 627 A1**

# EUROPEAN PATENT APPLICATION

(21) Application number: **92301525.9**

(22) Date of filing: **24.02.92**

(51) Int. Cl.5: **C12P 19/04, A61K 31/715**

(43) Date of publication of application:
**01.09.93 Bulletin 93/35**

(84) Designated Contracting States:
**AT BE CH DE DK ES FR GB IT LI NL**

(71) Applicant: **TAIYO KAGAKU Co., LTD.**
**9-5, Akahori-Shinmachi**
**Yokkaichi-shi Mie-ken 510(JP)**

(72) Inventor: **Aoyama, Nobuhiko, c/o Taiyo**
**Kagaku Co., Ltd.**
**9-5, Akahorishinmachi**
**Yokkaichi-shi, Mie-ken(JP)**
Inventor: **Hirata, Susumu, c/o Taiyo Kagaku**
**Co., Ltd.**
**9-5, Akahorishinmachi**
**Yokkaichi-shi, Mie-ken(JP)**
Inventor: **Takahashi, Hidehisa, c/o Taiyo**
**Kagaku Co., Ltd.**
**9-5, Akahorishinmachi**
**Yokkaichi-shi, Mie-ken(JP)**
Inventor: **Kadota, Noriaki, c/o Taiyo Kagaku**
**Co., Ltd.**
**9-5, Akahorishinmachi**
**Yokkaichi-shi, Mie-ken(JP)**

(74) Representative: **Marchant, James Ian et al**
**Elkington and Fife Prospect House 8**
**Pembroke Road**
**Sevenoaks, Kent TN13 1XR (GB)**

(54) **Limitedly enzyme-hydrolyzed polysaccharide and method for its production.**

(57) The present invention is directed to a limitedly enzyme-hydrolyzed polysaccharide obtained by reacting guar gum with $\beta$-mannanase, wherein not less than 80% of linear chains of mannose of said polysaccharide have a chain length of 30 to 200 units, a composition for biological regulatory function comprising an effective amount of said polysaccharide, and a method for its production. The polysaccharide of the present invention can be ingested without food palatability degradation even when added in large amounts and retains the high biological regulatory function of guar gum.

EP 0 557 627 A1

The present invention relates to a polysaccharide which has been limitedly hydrolyzed with enzyme and a method for its production.

Guar gum, one of the wide variety of high molecular polysaccharides, has been reported in many papers to have physiological effects such as significant reduction in blood or serum cholesterol level and improvement in constipation, and is now drawing increasing attention for its biological regulatory functions.

Guar gum is a polysaccharide obtained from the seeds of an annual leguminous plant. It is soluble in cold water, and since its aqueous solutions are particularly highly viscous (viscosity is about 3000 cps as of a 1% by weight aqueous solution) when compared among natural gum substances, it finds no uses other than thickening agents, water retaining agents, etc. for ice cream, soup, sauce and dressing, with the amount of its use limited to about 0.05 to 0.2% by weight of the food to which it is added. For this reason, even when it is intended to add guar gum to beverage or another food to utilize its biological regulatory function, the minimum amount required for the desired biological regulatory function cannot be taken when the amount of addition is small. If the amount of addition is increased to more than 0.2% by weight, for instance, the taste and palatability of the food is considerably deteriorated. Therefore, guar gum has not been used for the purpose of providing a particular function.

With the aim of utilizing this material, which is extremely viscous even at low concentrations of under 1% by weight, in beverage and other foods, various methods have been proposed to date. Examples of such proposed methods include the method of Japanese Patent Laid-Open No. 48230/1980, in which granules of guar gum having a diameter of 125 to 500 $\mu$ and a water content of 9 to 11% by weight are prepared as a gelation-free easy-to-take dispersion, the method of US Patent No. 4883788, in which guar gum is used in combination with carbonate etc. to generate gas by the action of gastric acid to improve the dispersibility, which makes it possible to lower the cholesterol level by oral administration, the method of Japanese Patent Laid-Open No. 175436/1984, in which guar gum is homogenized to obtain a 1.5% by weight aqueous solution having a viscosity of not more than 200 cps, which makes it possible to obtain mixed preparations of guar gum and food or feed, and the method of Japanese Patent Laid-Open No. 26993/1988, in which a plant tissue macerating enzyme is used to partially hydrolyze guar gum to lower its viscosity, which makes it possible to add a large amount of guar gum component to food and take it.

However, although these methods offer improvements in the easiness of drinking when guar gum is dispersed or dissolved in water, they have drawbacks such as gelation in the stomach and poor swallowability when taken as aqueous solutions. In the method of Japanese Patent Laid-Open No. 175436/1984, the viscosity is high at 200 cps when prepared as 1.5% by weight aqueous solution; to take the desired amount in beverage, the palatability is degraded or a large amount of water is required. In addition, the biological regulatory function is considerably degraded because the main and side chains of guar gum have been randomly cleaved. In the method of Japanese Patent Laid-Open No. 26993/1988, which uses a plant tissue macerating enzyme to obtain a hydrolyzate, the viscosity of 1% by weight aqueous solution is low at not more than 10 mPa • s, but increasing the concentration to about 10% by weight degrades the swallowability due to a rise in viscosity, which hampers intake of the amount required to obtain the desired biological regulatory function; therefore, this hydrolyzate cannot be used in large amounts in beverage. Moreover, because a plant tissue macerating enzyme is used, the main chain of guar gum is randomly cleaved and the chain length units become disintegrated so that the biological regulatory function is degraded. As stated above, viscosity reduction in high molecular polysaccharides results in functional deterioration.

As stated above, it is an object of the present invention to provide an enzyme-hydrolyzed polysaccharide which retains a high biological regulatory function of guar gum and which can be ingested with no degradation of food palatability even when added in large amounts.

It is another object of the present invention to provide a method for the production of an enzyme-hydrolyzed polysaccharide as described above.

The present inventors made investigations of enzyme-hydrolyzed polysaccharides and methods for their production, and found that a polysaccharide derived from guar gum which shows its function by oral administration without influence on the viscosity, texture, swallowability and other properties of the finished product can be obtained by reacting guar gum with $\beta$-mannanase to a limitedly hydrolyzed polysaccharide. The inventors made further investigations based on this finding, and developed the present invention. The present invention is hereinafter described in detail.

The starting material guar gum is a polysaccharide having a structure comprising $\beta$-(1,4)-D-mannopyranosyl units in the main chain and galactose branches bound thereto via $\alpha$-D(1,6) bond. The plant of its source or crude purification product thereof can also be used. $\beta$-mannanase is an enzyme which acts on the mannan of guar gum etc. to hydrolyze its $\beta$-(1,4) moiety. In the present invention, $\beta$-mannanase derived from microbes such as Aspergillus and Rhizopus can be used, and the method for its production is not

2

subject to limitation.

The limitedly enzyme-hydrolyzed polysaccharide of the present invention means that not less than 80% of linear chains of mannose have a chain length of 30 to 200 units, with preference given to 50 to 150 units from the viewpoint of retention of biological regulatory function. Also, the viscosity of 10% by weight aqueous solution of said polysaccharide with such a chain length is 20 to 5 cps at 25°C as determined using a Brookfield viscometer. If the polysaccharide is hydrolyzed to the extent that the chain length becomes less than 30 units and the viscosity becomes less than 5 cps, the biological regulatory function is degraded. If the degree of hydrolysis is such that the chain length exceeds 200 units and the viscosity exceeds 20 cps, the polysaccharide is difficult to use because the swallowability and palatability are poor when it is ingested as beverage. As stated above, by limited hydrolysis to the extent that not less than 80% of linear chains of mannose have a chain length of 30 to 200 units, it is possible to obtain a polysaccharide which retains the high biological regulatory function of guar gum without lowering food palatability even when it is added in large amounts. If the ratio of the linear chains of mannose falling in the above-mentioned range of chain length is less than 80%, the biological regulatory function lowers significantly in proportion to their content.

In the present invention, a biological regulatory function means a regulatory function which maintains a good physiological rhythm, including the prevention of, or recovery from, diseases such as hypertension and diabetes mellitus and the control of cancerous and other toxic substances.

For limitedly hydrolyzing guar gum with β-mannanase, guar gum and β-mannanase as produced by microbes such as Aspergillus niger are added to water, and the enzyme is allowed to act at 40 to 50°C for 30 to 70 hours until not less than 80% of the linear chains of mannose have a chain length of 30 to 200 units. After enzyme deactivation by heating at 90°C for 10 minutes, the impurities are removed by filtration etc., followed by deodorization and decoloration, whereby an enzyme-hydrolyzed polysaccharide derived from guar gum is obtained with no influence on the viscosity, texture, swallowability or other properties of the finished food product. It may be powdered as necessary by spray drying, freeze drying or drum drying, followed by pulverization. However, reaction time in this process is as long as 30 to 70 hours, and the productivity is poor, but this can be improved by adding xanthane gum in a ratio of not less than 0.1% by weight of β-mannanase (about 0.0001% of the substrate guar gum). In other words, xanthane gum is a high molecular polysaccharide which does not undergo hydrolytic action of said enzyme because it has no β-(1,4) bond and which is highly resistant to heat; it protects said enzyme and remarkably improves the heat tolerance. For this reaction, because it is not liable to be deactivated even when reacted at high temperature, it permits obtainment of the desired quality at 60 to 70°C with a reaction time of 5 to 20 hours, markedly saving reaction time.

Available hydrolytic methods include acid hydrolysis, alkali hydrolysis and hydrolysis using an oxidant such as chlorine. However, when guar gum is treated by these methods, its main and side chains are randomly cleaved; therefore, the biological regulatory function is considerably degraded, and the ratio of linear chains of mannose having a chain length of 30 to 200 units cannot be not less than 80% since the hydrolyzate thus obtained has an extremely wide linear mannose chain length distribution; it is impossible to produce a polysaccharide with the desired function.

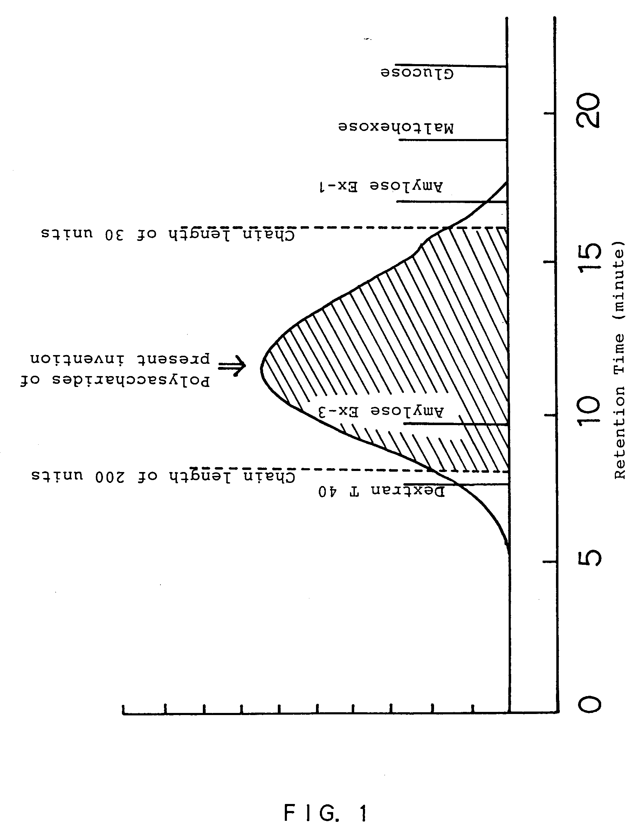

In the present invention, the chain length of linear chain of mannose means the number of mannose units constituting the main chain of guar gum, and the method for its determination is not subject to limitation. For example, the hydrolyzed polysaccharide sample is dissolved in water and subjected to gel filtration through a column of G3000 PWXL using a TOSO 803D model high performance liquid chromatograph (HPLC) and water as a mobile phase, followed by detection using a differential refractometer, whereby a graph as shown in Figure 1 is obtained. In this assay, glucose, maltohexose (produced by Seikagaku Kogyo), amylose Ex-1 (produced by Seikagaku Kogyo), amylose Ex-3 (produced by Seikagaku Kogyo) and dextran T40 (produced by Pharmacia), whose sugar chain length is 1 unit, 6 units, 18 units, 100 units and 250 units, respectively, are used as reference substances. Since these substances have a proportional relationship between elution peak position and logarithmically expressed chain length, the content of polysaccharides having a chain length of 30 to 200 units can be deduced from the area under the working curve. In other words, the number of mannose units is calculated on the assumption that 1 unit of glucose is equivalent to 1 unit of mannose. Figure 1 shows the elution pattern of polysaccharides and the elution peak position of reference substances by HPLC.

The enzyme-hydrolyzed polysaccharide of the present invention does not spoil the palatability of beverage and other foods because it shows no viscosity even when used in large amounts in beverage, and it retains the high biological regulatory function of guar gum. Its administration to humans or animals at given amounts makes it possible to obtain biological regulatory functions such as suppression of rise in cholesterol level, improvement in constipation, suppression of rise in blood sugar level and activation of enteric bacteria.

Although the amount of administration ensuring the effect of said polysaccharide is not subject to limitation, the same as with guar gum is applicable. In humans, for instance, the desired effect can be achieved by oral administration at 0.02 to 1.0 g/day per kg body weight; the polysaccharide may be used singly or in combination with other drugs, and may have any dosage form.

The percent ratio of addition of the polysaccharide is 5 to 20% in beverages or 5 to 30% in cakes, bread, cookies and other confectionery.

The present invention is hereinafter described in more detail by means of the following working examples, but the invention is not limited thereby.

In the examples given below, % means % by weight unless otherwise stated, and figures for cps were obtained from 10% solution at 25°C and 30 rpm using a Brookfield viscometer.

Example 1

To a culture solution of Rhizopus delemar, ammonium sulfate was added to precipitate enzyme protein, followed by recovery of this precipitate to yield a crude enzyme product. This precipitate was then dissolved in water and dialyzed, followed by impurity protein removal using ion exchange resin to yield a purified $\beta$-mannanase [R] (enzyme activity 1000 units/g, solid content 10%).

To 100 parts of a 5% guar gum solution, 40 units of the $\beta$-mannanase [R] thus obtained was added, followed by reaction at 40 °C for 41 hours. After completion of the reaction, 0.1 part of activated carbon was added and each solution was heated at 90 °C for 15 minutes to deactivate the enzyme, and impurities were filtered out. The resulting transparent solutions were powdered by spray drying to yield polysaccharides R.

To examine the physical properties of this polysaccharide R, gel filtration was conducted through a column of G3000 PWXL using a TOSO 803D model HPLC, followed by detection using a differential refractometer. Linear chains of mannose having a chain length of 30 to 200 units accounted for 88%, the viscosity was low at 13 cps, and the solution swallowability was good.

Example 2

To a culture of Aspergillus niger, ammonium sulfate was added to precipitate enzyme protein, followed by recovery of this precipitate to yield a crude enzyme product. This precipitate was then dissolved in water and dialyzed, followed by impurity protein removal using ion exchange resin to yield a purified $\beta$-mannanase [A] (enzyme activity 5000 units/g, solid content 10%).

To 100 parts of a 5% guar gum solution, 10 units of the $\beta$-mannanase [A] thus obtained was added, followed by reaction at 50°C for 36 hours. After completion of the reaction, 0.1 part of activated charcoal was added, and the enzyme was deactivated by heating at 90°C for 15 minutes, followed by filtering out the impurities. The resulting transparent solution was spray dried to yield a polysaccharide G.

This polysaccharide G was assayed in the same manner as in Example 1. Linear chains of mannose having a chain length of 30 to 200 units accounted for 92%, the viscosity was low at 9 cps, and the solution swallowability was good.

Example 3

Reaction was carried out at 70°C for 5, 10 and 20 hours in each of two experimental plots. In Experimental Plot X, 40 units of the $\beta$-mannanase [R] obtained in Example 1 was added to 100 parts of a 5% guar gum solution. In Experimental Plot Y, 0.00002 part of xanthane gum (0.5% of the enzyme weight) was added to 40 units of the same $\beta$-mannanase. After completion of the reaction, the same treatment as in Example 1 was carried out to yield 5-, 10- and 20-hr reacted polysaccharides in each of Experimental Plots X and Y. The polysaccharides thus obtained had the viscosities shown in Table 1.

Table 1

| Reaction time (hour) | Experimental plot X β-mannanase | Experimental plot Y β-mannanase + xanthane gum |
|---|---|---|
| 0 | - | - |
| 5 | - | 194 cps |
| 10 | - | 17 cps |
| 20 | - | 6 cps |
| - : not determined due to gel formation | | |

As seen in Table 1, viscosity reduction could not be noted even after 20 hours of reaction because determination was impossible due to gel formation in Experimental Plot X, while the viscosity lowered gradually as the reaction time increased, with the desired viscosity obtained only in 10 hours, in Experimental Plot Y.

This finding demonstrates that at a high reaction temperature of 70°C, the enzyme is deactivated when it is used singly as in Experimental Plot X, while the enzyme is not deactivated, the desired viscosity can be rapidly obtained, and the production process time can be remarkably shortened when it is used in combination with xanthane gum added before the enzyme reaction as in Experimental Plot Y.

Test Example 1

To 100 parts of a 5% guar gum solution, 10 units of the β-mannanase enzyme obtained in Example 2 was added, followed by reaction at 45°C for 10, 20, 35, 45, 65 or 80 hours. After completion of the reaction, each solution was heated at 90°C for 15 minutes to deactivate the enzyme, and impurities were filtered out. The resulting transparent solutions were powdered by spray drying to yield polysaccharides A, B, C, D, E and F.

The physical properties of these polysaccharides thus obtained were examined, and their 10% aqueous solutions were evaluated by a sensory test. The results are shown in Table 2.

Table 2

| Polysaccharides | Reaction time (hour) | Chain length | | | Viscosity (cps) | Sensory test |
|---|---|---|---|---|---|---|
| | | >200unit (%) | 30~200 unit(%) | <30unit (%) | | |
| A | 10 | 96 | 4 | — | 990 | extremely high viscosity |
| B | 20 | 47 | 53 | — | 45 | slight viscosity |
| C | 35 | 16 | 83 | 1 | 18 | not bad swallowability |
| D | 45 | 2 | 96 | 2 | 11 | good swallowability |
| E | 65 | 1 | 81 | 18 | 7 | good swallowability |
| F | 80 | — | 45 | 55 | 3 | good swallowability |

viscosity:10% by weight aqueous solution of each polysaccharide

As seen in Table 2, it is evident that the maximum critical point of chain length for freedom from drinking discomfort in beverage use is 200 units, since the aqueous solutions of the polysaccharides C, D, E and F had good swallowability while those of the polysaccharides A and B, both of which had a high ratio of chains having a chain length exceeding 200 units, had poor evaluation in the sensory test.

Test Example 2

To assess the suppressive effect on rise in cholesterol level, an animal experiment in rats was conducted using the polysaccharides A, B, C, D, E and F obtained in Test Example 1.

(a) Experimental foods: The compositions of the animal foods used in the experiment are shown in Table 3.

Table 3

|  | Control | Food A | Food B | Food C | Food D | Food E | Food F |
|---|---|---|---|---|---|---|---|
| Casein | 22 | 22 | 22 | 22 | 22 | 22 | 22 |
| Corn oil | 1 | 1 | 1 | 1 | 1 | 1 | 1 |
| Lard | 10 | 10 | 10 | 10 | 10 | 10 | 10 |
| Mixture of salts | 4 | 4 | 4 | 4 | 4 | 4 | 4 |
| Mixture of vitamin | 1 | 1 | 1 | 1 | 1 | 1 | 1 |
| Corn starch | 61.6 | 59.1 | 59.1 | 59.1 | 59.1 | 59.1 | 59.1 |
| Cholesterol | 0.4 | 0.4 | 0.4 | 0.4 | 0.4 | 0.4 | 0.4 |
| Polysaccharide A | — | 2.5 | — | — | — | — | — |
| Polysaccharide B | — | — | 2.5 | — | — | — | — |
| Polysaccharide C | — | — | — | 2.5 | — | — | — |
| Polysaccharide D | — | — | — | — | 2.5 | — | — |
| Polysaccharide E | — | — | — | — | — | 2.5 | — |
| Polysaccharide F | — | — | — | — | — | — | 2.5 |

(b) Experimental animals: Male Wistar rats weighing about 50 g at 4 weeks of age were used. After acclimation with commercially available solid food for 4 to 5 days, animals were allotted to groups each of which comprised 6 animals. The animals had free access to the experimental foods and water. They were used after 14 days of rearing.

(c) Method of assay: After the rats were fasted overnight, cardiac blood was collected under nembutal anesthesia, and then the liver was excised. After blood centrifugation, plasma was collected and assayed for cholesterol, and the liver, after weighing, was assayed for cholesterol.

(d) Results: The experimental results are shown in Table 4.

Table 4

| Sample | Serum cholesterol | Liver cholesterol |
|--------|-------------------|-------------------|
| control | 62.3 ± 3.6 | 4.9 ± 0.5 |
| food A | 40.3 ± 1.2 | 2.2 ± 0.4 |
| food B | 41.5 ± 3.3 | 2.3 ± 0.3 |
| food C | 41.7 ± 2.6 | 2.4 ± 0.1 |
| food D | 41.3 ± 2.8 | 2.4 ± 0.2 |
| food E | 46.3 ± 3.1 | 3.1 ± 0.2 |
| food F | 60.0 ± 3.9 | 4.5 ± 0.2 |

As seen in Table 4, the rise in cholesterol level in the rat groups fed with a food containing the polysaccharide A, B, C, D or E was clearly suppressed, while the polysaccharide F, whose major component has a linear mannose chain length of less than 30 units, had a considerably lower suppressive effect; therefore, it is evident that the chain length should be at least not less than 30 units to retain the high biological regulatory function. Also, the cholesterol suppressive effect is reported to weaken with the degree of hydrolysis, which agrees well with the finding that the polysaccharide F, which has a viscosity of 3 cps, had a lower effect.

Test Example 3

To assess the improving effect on constipation, an animal experiment in rats was conducted using the polysaccharides A, B, C, D, E and F obtained in Example 2.

(a) Experimental foods: The compositions of the experimental foods used in the experiment are shown in Table 3. The foods were supplemented with 1% chromic oxide as a marker (green).

(b) Experimental animals: The same rats as in Test Example 2 were used.

(c) Experimental method: The rats were fasted for 24 hours. The interval between the time when the rats started to eat and the time when feces with the same color as the marker's color were excreted was measured for digestive tract passage time.

(d) Results: The experimental results for digestive tract passage time are shown in Table 5.

Table 5

| Sample | Digestive tract passage time (hour) |
|--------|-------------------------------------|
| control | 15.2 ± 1.7 |
| food A | 9.2 ± 1.6 |
| food B | 9.4 ± 1.5 |
| food C | 9.4 ± 1.7 |
| food D | 9.6 ± 1.3 |
| food E | 10.4 ± 1.1 |
| food F | 14.0 ± 0.8 |

As seen in Table 5, digestive tract passage time improved clearly in the rat groups fed with a food containing the polysaccharide A, B, C, D or E, while the polysaccharide F, whose main component has a linear mannose chain length of less than 30 units, had almost no such effect; it is evident that the chain length should be at least not less than 30 units to retain the high biological regulatory function.

Test Example 4

To assess the suppressive effect on the rise in blood sugar level, an animal experiment in rats was conducted using the polysaccharides A, B, C, D, E and F obtained in Example 2.

(a) Experimental animals: The same rats as in Test Example 2 were used.

(b) Experimental method: Animals were allotted to 1 control group treated with a 10% glucose solution alone and groups treated with a glucose solution containing 10% of each the polysaccharides A, B, C, D, E and F. Each solution was administered at 125 mg per 100 g of body weight using a gastric sound, and blood was sampled via the tail vein and assayed for blood sugar level over time.

(c) Results: The results for blood sugar level are shown in Table 6.

Table 6

| Time (min) | Control | Food A | Food B | Food C | Food D | Food E | Food F |
|---|---|---|---|---|---|---|---|
| 0 | 80 | 80 | 80 | 80 | 80 | 80 | 80 |
| 15 | 240 | 140 | 142 | 145 | 144 | 160 | 190 |
| 30 | 230 | 145 | 148 | 150 | 151 | 187 | 197 |
| 60 | 175 | 142 | 143 | 145 | 147 | 162 | 170 |
| 120 | 120 | 126 | 125 | 127 | 126 | 128 | 118 |
| 180 | 110 | 100 | 103 | 105 | 109 | 113 | 111 |

As seen in Table 6, the rapid rise in blood sugar level was clearly suppressed in the rat groups fed with a food containing the polysaccharide A, B, C, D or E, while the polysaccharide F, whose main component has a linear mannose chain length of less than 30 units, had a considerably lower suppressive effect; therefore, it is evident that the chain length should be at least not less than 30 units to retain the high biological regulatory function.

Test Example 5

To assess the improving effect on enteric bacterial flora, a bacterial proliferation experiment for the useful enteric bacteria Bifidobacterium longum and Lactobacillus acidophilus was conducted using the polysaccharides A, B, C, D, E and F obtained in Example 1.

(a) Culture medium: The composition of the culture medium used in the experiment is shown in Table 7.

Table 7

| | |
|---|---|
| Bact • liver(DIFCO) decoction | 1000 ml |
| Proteosepeptone(DIFCO) | 10 g |
| Tryptycase(BBL) | 5 g |
| Yeast extract(DIFCO) | 3 g |
| Tween 80 | 1 g |
| L-cysteine • HCl • $H_2O$ | 0.2 g |
| Polysaccharide | 5.0 g |
| pH | 7.0 |

(b) Experimental method: Cells of each enteric bacterium were inoculated to a culture medium containing 0.5% of each polysaccharide. After anaerobic cultivation at 37°C for 48 hours, bacterial proliferation was measured on the basis of absorbance at 600 nm. The percent ratio relative to the absorbance obtained when 0.5% glucose was added as a sugar source was used to express the degree of proliferation.

(c) Results: The results for enteric bacterial proliferation promoting effect are shown in Table 8.

9

Table 8

| Sample | Bifidobacterium longum | Lactobacillus acidophilus |
|---|---|---|
| glucose | + + | + + |
| food A | - | - |
| food B | - | ± |
| food C | + | + |
| food D | + + | + + |
| food E | + + | + + |
| food F | + + | + + |

+ + : 51~100 + : 11~50 ± : 1~10 - : 0

As seen in Table 8, the culture medium supplemented with the polysaccharide C, D, E or F had a proliferation promoting effect on the useful enteric bacterium in the genus Bifidobacterium and genus Lactobacillus, while the polysaccharides A and B, whose major component has a linear mannose chain length exceeding 200 units, had almost no proliferation promoting effect; therefore, it is evident that the chain length should be not more than 200 units to obtain the desired effect.

The results in these Test Examples demonstrate that a polysaccharide which can be ingested without food palatability degradation even when added in large amounts and which retains the high biological regulatory function of guar gum can be obtained by limitedly hydrolyzing guar gum with enzyme so that the chain length of linear chain of mannose is 30 to 200 units and the 10% aqueous solution viscosity is 20 to 5 cps at 25°C.

**Claims**

1. A limitedly enzyme-hydrolyzed polysaccharide obtained by reacting guar gum with $\beta$-mannanase, wherein not less than 80% of linear chains of mannose of said polysaccharide have a chain length of 30 to 200 units.

2. A limitedly enzyme-hydrolyzed polysaccharide according to claim 1, wherein a viscosity of 10% by weight aqueous solution of said polysaccharide is 20 to 5 cps at 25°C as determined using a Brookfield viscometer.

3. A composition for biological regulatory function comprising an effective amount of a limitedly enzyme-hydrolyzed polysaccharide obtained by reacting guar gum with $\beta$-mannanase, wherein not less than 80% of linear chains of mannose of said polysaccharide have a chain length of 30 to 200 units.

4. A composition according to claim 3, wherein said function is the suppressive effect on rise in cholesterol level.

5. A composition according to claim 3, wherein said function is the improving effect on constipation.

6. A composition according to claim 3, wherein said function is the suppressive effect on the rise in blood sugar level.

7. A composition according to claim 3, wherein said function is the improving effect on enteric bacterial flora.

8. A process of producing a limitedly enzyme-hydrolyzed polysaccharide, comprising the step of reacting guar gum with $\beta$-mannanase so that not less than 80% of linear chains of mannose of said polysaccharide is 30 to 200 units.

9. A process according to claim 8, wherein xanthane gum is further added in a ratio of not less than 0.1% by weight of $\beta$-mannanase at the limitedly enzyme-hydrolyzation.

F I G. 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | PATENT ABSTRACTS OF JAPAN<br>vol. 14, no. 566 (C-789)(4509) 17 December  1990<br>& JP-A-2 248 401 ( GODO SHIYUSEI K.K. ) 4 October 1990 | 1,3,5,8 | C12P19/04<br>A61K31/715 |
| Y | * abstract * | 4,6 | |
| X | CHEMICAL ABSTRACTS, vol. 93, no. 21,<br>1980, Columbus, Ohio, US;<br>abstract no. 202707S, 'Low-viscosity guar gum'<br>page 528 ;<br>* abstract *<br>& JP-A-55 027 797 (TOYOBO CO., LTD.) 23 July 1980 | 1,8 | |
| X | CHEMICAL ABSTRACTS, vol. 82, no. 3,<br>20 January  1975, Columbus, Ohio, US;<br>abstract no. 15548B, 'Low viscosity guar gum'<br>page 397 ;<br>* abstract *<br>& JP-A-49 018 235 (FUKUMOTO, JUICHIRO ET AL.) 8 May 1974 | 1,8 | |
| D,Y | PATENT ABSTRACTS OF JAPAN<br>vol. 9, no. 28 (C-264)(1751) 6 February 1985<br>& JP-A-59 175 436 ( SHIYOUWA SANGYO K.K. ) 4 October 1984<br>* abstract * | 4 | TECHNICAL FIELDS SEARCHED (Int. Cl.5 )<br><br>C12P<br>C12N<br>A61K |
| Y | EP-A-0 080 673 (BOEHRINGER MANNHEIM GMBH)<br>* page 1, line 1 - page 2, line 10 * | 6 | |

The present search report has been drawn up for all claims

| Place of search | Date of completion of the search | Examiner |
|---|---|---|
| THE HAGUE | 14 SEPTEMBER 1992 | MONTERO LOPEZ B. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
......................................................................................................
& : member of the same patent family, corresponding
    document

EPO FORM 1503 03.82 (P0401)